(19) **Europäisches Patentamt**

**European Patent Office**

**Office européen des brevets**

(11) **EP 1 282 417 B1**

(12) **EUROPÄISCHE PATENTSCHRIFT**

(45) Veröffentlichungstag und Bekanntmachung des Hinweises auf die Patenterteilung:
**26.01.2005   Patentblatt 2005/04**

(51) Int Cl.[7]: **A61K 31/415**, C07D 233/88

(21) Anmeldenummer: 01931189.3

(86) Internationale Anmeldenummer:
**PCT/AT2001/000138**

(22) Anmeldetag: **14.05.2001**

(87) Internationale Veröffentlichungsnummer:
**WO 2001/087300 (22.11.2001 Gazette 2001/47)**

(54) **CREATININ-BIOSENSOR**

CREATININE BIOSENSOR

BIODETECTEUR DE CREATININE

(84) Benannte Vertragsstaaten:
**DE FR GB**

(30) Priorität: **16.05.2000  AT  8532000**

(43) Veröffentlichungstag der Anmeldung:
**12.02.2003   Patentblatt 2003/07**

(73) Patentinhaber: **F. Hoffmann-La Roche AG
4070 Basel (CH)**

(72) Erfinder: **SCHAFFAR, Bernhard, Peter, Harald
A-8045 Graz (AT)**

(74) Vertreter: **Schwarz, Albin, Dr. et al
Kopecky & Schwarz
Patentanwälte
Wipplingerstrasse 32/22
1010 Wien (AT)**

(56) Entgegenhaltungen:
**FR-A- 2 682 765          US-A- 5 466 575**

- **TSUCHIDA T ET AL: "MULTI-ENZYME MEMBRANE ELECTRODES FOR DETERMINATION OF CREATININE AND CREATINE IN SERUM" QUALITY CONTROL CLIN. CHEM. TRANSACTIONS INTERNATIONAL SYMPOSIUM, XX, XX, Bd. 29, Nr. 1, 1983, Seiten 51-55, XP001008686**

**Beschreibung**

Die Erfindung betrifft ein Verfahren zur Herstellung von Biosensoren mit mindestens zwei Enzymen zur amperometrischen Bestimmung enzymatisch abbaubarer Stoffe in biologischen Flüssigkeiten; wobei die Enzyme an einer Arbeitselektrode immobilisiert werden. Die Erfindung betrifft weiters einen Biosensor, insbesondere zur Bestimmung von Creatinin.

Die Bestimmung enzymatisch abbaubarer Stoffe, wie Creatinin, Glucose, etc., mittels Sensoren in biologischen Flüssigkeiten, zum Beispiel in Blut, Urin, Plasma, Serum und Liquor, erfolgt vorzugsweise über Biosensoren mit immobilisierten Enzymen. In der Literatur sind mehrere elektrochemische und photometrische Verfahren zur Bestimmung dieser Stoffe bekannt.

So kann beispielsweise über das Enzym Creatinine Deiminase mit nachfolgender Bestimmung des Ammoniumgehalts Creatinin potentiometrisch bestimmt werden. Ein anderes Verfahren besteht darin, die Creatininkonzentration über eine Enzymkaskade unter Verwendung der Enzyme Creatininase, Creatinase und Sarkosinoxidase zu bestimmen, wobei letztendlich Wasserstoffperoxid ($H_2O_2$) an einer amperometrischen Elektrode gemessen wird.

Die Erfindung bezieht sich auf ein Verfahren zur Herstellung von Biosensoren, die nach dem letztgenannten Prinzip arbeiten. Hierzu müssen die Enzyme coimmobilisiert werden, um die Umsetzung von Creatinin in das amperometrisch detektierbare Molekül Wasserstoffperoxid zu ermöglichen. Die Umsetzung von Creatinin zu Wasserstoffperoxid erfolgt nach den folgenden Reaktionsschritten:

$$\text{Creatinin} + H_2O \qquad \text{—— Creatininase} \rightarrow \qquad \text{Creatin}$$

$$\text{Creatin} + H_2O' \qquad \text{—— Creatinase} \rightarrow \qquad \text{Sarkosin} + \text{Harnstoff}$$

$$\text{Sarkosin} + O_2 + H_2O \qquad \text{—— Sarkosinoxidase} \rightarrow \qquad \text{Glycin} + \text{Formaldehyd} + H_2O_2$$

An der amperometrischen Elektrode wird Wasserstoffperoxid anodisch bei -350 mV gegen Ag/AgCl oxidiert. Der dabei fließende Strom ist der Creatininkonzentration proportional.

$$H_2O_2 \qquad \text{-350 mV} \qquad \rightarrow \qquad \text{2 Protonen} + \text{2 Elektronen} + O_2$$

Der bei der Elektrodenreaktion rückgewonnene Sauerstoff wird zur Oxidation des Sarkosins weiterverwendet.

Im Stand der Technik sind mehrere Arten zur Immobilisierung der drei verwendeten Enzyme bekannt. Gemäß T. Tsuchida, K. Yoda, Clin. Chem. 29/1, S. 51, 1983, werden alle drei Enzyme mit Glutardialdehyd vernetzt.

Diese Methode der Immobilisierung besitzt jedoch den Nachteil, daß mit einem derartig hergestellten Biosensor nur eine geringe Signalhöhe erreicht wird, d.h. nur eine geringe Stromänderung feststellbar ist, da die derart immobilisierte Sarkosinoxidase fast ihre gesamte Aktivität verliert. Eine möglichst große Signalhöhe ist jedoch speziell bei der Creatininbestimmung besonders wichtig, weil die Creatininkonzentration vor allem in Blut äußerst niedrig ist (ca. 50 μM) und zudem die Creatinase nur in sehr geringen spezifischen Aktivitäten (max. 20 iu/mg) erhältlich ist. Weiters weist ein derart immobilisierter Sensor hohe Ansprechzeiten auf.

In der US-A - 5,466,575 ist ein Verfahren beschrieben, bei dem Sarkosinoxidase und Creatininase in einem photovernetzbaren Fisch-Gel immobilisiert und anschließend mit Creatinase in einem filmbildenden Polyvinylacetat-co-vinylalkohol-Latex überschichtet werden.

Nachteilig hierbei ist jedoch die aufwendige Photovemetzung, die eine einfache Herstellung des Biosensors unmöglich macht.

Evtugyn et al. (Analyst 121 (1996), 1911-1915) beschreiben den Einfluss nicht-ionogener oberflächenaktiver Stoffe auf das Verhalten und die Empfindlichkeit von potentiometrischen Cholinesterase-Biosensoren zur Bestimmung reversibler und irreversibler Enzym-Inhibitoren. So können die zur untersuchten Probe hinzugefügten oberflächenaktiven Substanzen in Abhängigkeit von der Konzentration und des Enzymträgermaterials als reversible Inhibitoren wirken und u.a. die Nachweisgrenzen fiir irreversible Inhibitoren senken.

Die Erfindung stellt sich die Aufgabe, ein Verfahren der eingangs genannten Art bereitzustellen, das die oben genannten Nachteile und Schwierigkeiten überwindet. Insbesondere soll das erfindungsgemäße Verfahren eine einfache Herstellung eines Biosensors ermöglichen, mit welchem sowohl kurze Ansprechzeiten als auch große Signalhöhen erzielbar sind. Insbesondere soll die Immobilisierung der Enzyme bei Raumtemperatur möglich sein.

Diese Aufgabe wird erfindungsgemäß dadurch gelöst, daß ein Enzym mit einem oder mehreren oberflächenaktiven Stoffen in wäßriger Lösung auf die Arbeitselektrode aufgebracht und trocknen gelassen wird und das zumindest zweite Enzym in einem nachfolgenden Schritt darauf chemisch immobilisiert wird.

Für die Zwecke der vorliegenden Beschreibung und Patentansprüche soll der Begriff "oberflächenaktive Stoffe" Stoffe, die oberflächenaktive Eigenschaften besitzen, wie Detergenzien und Alkohole, beispielsweise Glycerin, umfassen.

Vorzugsweise werden als oberflächenaktive Stoffe Polyalkohole und/oder Detergenzien, bevorzugt nichtionische Tenside, eingesetzt.

Es wurde festgestellt, daß durch diese Zusätze der gemessene Strom im Vergleich zu Biosensoren mit drei gleich immobilisierten Enzymen um ca. Faktor 40 erhöht wird.

Das zumindest zweite Enzym wird zweckmäßig durch Cross-linking, kovalente Bindung oder Matrixeinschluß immobilisiert. Vorzugsweise wird die Immobilisierung mittels Glutardialdehyd bewirkt.

In einer bevorzugten Ausführungsform wird nach der Immobilisierung der Enzyme eine Deckmembran aufgebracht. Eine solche Membran, z.B. aus Nafion, PVC-Copolymer oder Celluloseacetat, erhöht vorteilhaft die Linearität der Sensoren und bewirkt zusätzlich eine Reduktion von Interferenzeinflüssen.

Ein erfindungsgemäßer Biosensor, welcher eine Arbeits-, eine Referenz- und eine Gegenelektrode aufweist und dessen Enzyme mittels des erfindungsgemäßen Verfahrens immobilisiert wurden, ist dadurch gekennzeichnet, daß die Referenzelektrode eine Ag/AgCl-Elektrode ist, die Gegenelektrode eine Kohlenstoffelektrode ist, die Arbeitselektrode aus Kohlenstoff, Metall, Metalloxiden oder einer Mischung aus Kohlenstoff und Metall oder Metalloxiden besteht und die Elektroden auf einem nicht leitenden Substrat aufgebracht sind.

Insbesondere ist ein erfindungsgemäßer Biosensor zur Bestimmung von Creatinin dadurch gekennzeichnet, daß an der Arbeitselektrode Sarkosinoxidase adsorbiert ist und darauf Creatininase und Creatinase immobilisiert sind.

In einer bevorzugten Ausgestaltung ist der Biosensor aus zwei Dreielektrodensystemen aufgebaut, welches erste Elektrodensystem mit den Enzymen Creatininase, Creatinase und Sarkosinoxidase zur Bestimmung der Summe von Creatinin und Creatin und welches zweite Elektrodensystem mit den Enzymen Creatinase und Sarkosinoxidase zur Bestimmung von Creatin dient, wobei das Ergebnis des zweiten Elektrodensystems von jenem des ersten zur Bestimmung von Creatinin abgezogen wird.

Vorteilhaft umfaßt der Biosensor ein weiteres Elektrodensystem, welches zur Eliminierung von elektrochemischen Interferenzen dient.

Die Erfindung wird nachstehend durch die folgenden Beispiele weiter veranschaulicht:

Beispiel 1

Beispiel 1 zeigt die Verbesserung der Signalhöhe durch Erhöhung der Sarkosinoxidase-Aktivität bei erfindungsgemäßem Zusatz von oberflächenaktiven Stoffen im Vergleich zum Stand der Technik.

Sarkosinoxidase wurde gelöst in Wasser (Stand der Technik) sowie in Wasser unter Zusatz von wasserlöslichen, oberflächenaktiven Komponenten (im vorliegenden Fall Glycerin sowie drei nichtionische Tenside) auf den amperometrischen Basissensor getropft und bei Raumtemperatur trocknen gelassen. Nach erfolgter Polarisation der Elektrode wurde der Strom auf 1 mM Sarkosin gemessen. Das Ergebnis der Messung ist in Tabelle 1 angeführt.

Tabelle 1

| Sarkosinoxidase (SOx) | Zusatz | Strom auf 1 mM Sarkosin |
|---|---|---|
| 54,2 mg SOx in 0,5 ml $H_2O$ | keiner | 2 nA |
| 54,2 mg SOx in 0,5 ml $H_2O$ | 5,0 % Glycerin | 80 nA |
| 54,2 mg SOx in 0,5 ml $H_2O$ | 0,5 % Tween 20 | 70 nA |
| 54,2 mg SOx in 0,5 ml $H_2O$ | 0,5 % Triton X100 | 90 nA |
| 54,2 mg SOx in 0,5 ml $H_2O$ | 0,5 % Brij 35 | 85 nA |

Es zeigte sich, daß der Strom auf 1 mM Sarkosin durch Detergenzien bzw. Glycerin um ca. Faktor 40 erhöht werden konnte.

Diese enorme Stromerhöhung wird offenbar dadurch bewirkt, weil das Enzym beim Trocknen durch die Zusätze optimal geschützt wird und weil die oberflächenaktiven Eigenschaften der Zusätze zu einem besseren und innigeren Verbund mit der porösen Struktur der Wasserstoffperoxidelektrode führen.

Beispiel 2

Beispiel 2 zeigt die Verbesserung der Signalhöhe sowie die Verkürzung der Ansprechzeit eines erfindungsgemäßen Biosensors im Vergleich zu einem gemäß Stand der Technik (T. Tsuchida) hergestellten Biosensor.

Es wurden zwei vollständige Creatininsensoren hergestellt, wobei beim ersten Sensor alle drei Enzyme zusammen mit Glutardialdehyd vernetzt wurden und beim zweiten Sensor Sarkosinoxidase mit Tween 20 zuerst auf die Basiselektrode aufgebracht wurde und danach Creatininase und Creatinase mit Glutardialdehyd darauf immobilisiert wurden. Die resultierenden Ströme bzw. Ansprechzeiten zur Messung von Creatinin bzw. Sarkosin sind in Tabelle 2 angegeben.

Tabelle 2

| Sarkosinoxidase | Strom auf 1 mM Creatinin | Strom auf 1 mM Sarkosin | Ansprechzeit (T90) |
|---|---|---|---|
| In Glutardialdehyd (Sensor 1) | 120 nA | 140 nA | 80 s |
| In Tween 20 (Sensor 2) | 420 nA | >500 nA | 10 s |

Es wurde mittels der erfindungsgemäßen Immobilisierung der Enzyme ein um ein Vielfaches höherer Strom gemessen. Die Ansprechzeit des erfindungsgemäß hergestellten Sensors war ebenfalls deutlich kürzer als jene des im Stand der Technik bekannten Sensors.

Beispiel 3

In Beispiel 3 ist die Herstellung eines erfindungsgemäßen Creatinin-Biosensors beschrieben.

Auf einem elektrisch nicht leitenden Substrat aus Kunststoff oder Keramik werden mittels Siebdruckverfahren Ag-Leiterbahnen für Referenz-, Gegen- und Arbeitselektroden gedruckt. Die Referenzelektrode wird zumindest im Sensorbereich aus einer Ag/AgCl-Paste hergestellt. Die Gegenelektrode wird im Meßbereich mit einer Schicht Carbonpaste bedruckt. Mit derselben Carbonpaste wird die Ag-Leiterbahn der Arbeitselektrode in den Meßbereich verlängert. Im Meßbereich der Arbeitselektrode wird eine Mischung aus 5 % Mangandioxid in Carbonpaste als Arbeitselektrode gedruckt. Im Anschluß wird das gesamte System mit Ausnahme der später mit Flüssigkeit zu kontaktierenden Elektrodenspots und der dem Signalabgriff dienenden Leiterbahnen mehrfach mit einem Isolationslack überzogen. Danach wird die Arbeitselektrode mit Sarkosinoxidase in Tween 20 - Lösung aufgetropft und trocknen gelassen. Hierauf werden die anderen Enzyme mittels Glutardialdehyd immobilisiert. Zur Erhöhung der Linearität und zur Verminderung von störenden Einflüssen wird eine Deckmembran aufgebracht.

Um Creatinin interferenzfrei bestimmen zu können, sind zumindest zwei Dreielektrodensysteme nötig; ein System mit den Enzymen Creatininase, Creatinase und Sarkosinoxidase, welches Creatinin und Creatin bestimmt, sowie ein weiteres mit den Enzymen Creatinase und Sarkosinoxidase, mit welchem Creatin bestimmt werden kann. Da Creatin im Blut als Interferent vorkommt, muß der gemessene Creatinwert vom Meßwert der Creatininelektrode abgezogen werden, der sich aus Creatin und Creatinin zusammensetzt.

Um weitere elektrochemische Interferenzen zu eliminieren, kann ein drittes Elektrodensystem mit immobilisierter Sarkosinoxidase allein (Creatin wird durch ein inaktives Protein, z.B. Albumin ersetzt) verwendet werden.

**Patentansprüche**

1. Verfahren zur Herstellung von Biosensoren mit mindestens zwei Enzymen zur amperometrischen Bestimmung enzymatisch abbaubarer Stoffe, wie Creatinin, in biologischen Flüssigkeiten, wobei die Enzyme an einer Arbeitselektrode immobilisiert werden, **dadurch gekennzeichnet, daß** ein Enzym mit einem oder mehreren oberflächenaktiven Stoffen in wäßriger Lösung auf die Arbeitselektrode aufgebracht und trocknen gelassen wird und das zumindest zweite Enzym in einem nachfolgenden Schritt darauf chemisch immobilisiert wird.

2. Verfahren nach Anspruch 1, **dadurch gekennzeichnet, daß** als oberflächenaktive Stoffe Polyalkohole und/oder Detergenzien, vorzugsweise nichtionische Tenside, eingesetzt werden.

3. Verfahren nach Anspruch 1 oder 2, **dadurch gekennzeichnet, daß** das zumindest zweite Enzym durch Crosslinking, kovalente Bindung oder Matrixeinschluß immobilisiert wird.

4. Verfahren nach Anspruch 3, **dadurch gekennzeichnet, daß** das zumindest zweite Enzym mittels Glutardialdehyd immobilisiert wird.

5. Verfahren nach einem der Ansprüche 1 bis 4, **dadurch gekennzeichnet, daß** nach der Immobilisierung eine Deckmembran aufgebracht wird.

6. Biosensor mit Arbeits-, Referenz- und Gegenelektrode, hergestellt mittels des Verfahrens nach einem der Ansprüche 1 bis 5, **dadurch gekennzeichnet, daß** die Referenzelektrode eine Ag/AgCl-Elektrode und die Gegenelektrode eine Kohlenstoffelektrode ist und die Arbeitselektrode aus Kohlenstoff, Metall, Metalloxiden oder einer Mischung aus Kohlenstoff und Metall oder Metalloxiden besteht, wobei die Elektroden auf einem nicht leitenden Substrat aufgebracht sind.

7. Biosensor nach Anspruch 6 zur Bestimmung von Creatinin, **dadurch gekennzeichnet, daß** an der Arbeitselektrode Sarkosinoxidase adsorbiert ist und darauf Creatininase und Creatinase immobilisiert sind.

8. Biosensor nach Anspruch 7, **dadurch gekennzeichnet, daß** er aus zwei Dreielektrodensystemen aufgebaut ist, welches erste Elektrodensystem mit den Enzymen Creatininase, Creatinase und Sarkosinoxidase zur Bestimmung der Summe von Creatinin und Creatin und welches zweite Elektrodensystem mit den Enzymen Creatinase und Sarkosinoxidase zur Bestimmung von Creatin dient, wobei die beiden Ergebnisse zur Bestimmung von Creatinin subtrahiert werden.

9. Biosensor nach Anspruch 8, welcher ein weiteres Elektrodensystem umfaßt, das zur Eliminierung von elektrochemischen Interferenzen dient.

**Claims**

1. A method for producing biosensors comprising at least two enzymes, for the amperometric determination of enzymatically degradable substances, such as creatinine, in biological liquids, the enzymes being immobilized on a working electrode, **characterized in that** an enzyme together with one or more surface-active substances in an aqueous solution is applied on the working electrode and is allowed to dry, and the at least second enzyme is chemically immobilized thereupon in a subsequent step.

2. A method according to claim 1, **characterized in that** polyalcohols and/or detergents, preferably non-ionic tensides, are used as surface-active substances.

3. A method according to claim 1 or 2, **characterized in that** the at least second enzyme is immobilized by means of crosslinking, covalent binding or matrix inclusion.

4. A method according to claim 3, **characterized in that** the at least second enzyme is immobilized by means of glutardialdehyde.

5. A method according to any of claims 1 to 4, **characterized in that** a cover membrane is applied after immobilization.

6. A biosensor comprising a working, a reference and a counter electrode, produced by means of the method according to any of claims 1 to 5, **characterized in that** the reference electrode is an Ag/AgCl electrode and the counter electrode is a carbon electrode and the working electrode consists of carbon, metal, metal oxides or a mixture of carbon and metal or metal oxides, the electrodes being applied on a nonconducting substrate.

7. A biosensor according to claim 6 for the determination of creatinine, **characterized in that** sarcosine oxidase is adsorbed on the working electrode and creatininase and creatinase are immobilized thereupon.

8. A biosensor according to claim 7, **characterized in that** it is made up of two three-electrodes systems, the first electrode system comprising the enzymes creatininase, creatinase and sarcosine oxidase and serving for the determination of the sum of creatinine and creatine and the second electrode system comprising the enzymes creatinase and sarcosine oxidase and serving for the determination of creatine, whereby the two results are subtracted for the determination of creatinine.

9. A biosensor according to claim 8 which comprises a further electrode system serving for the elimination of electrochemical interferences.

**Revendications**

**1.** Procédé pour la préparation de biocapteurs avec au moins deux enzymes pour la détection ampérométrique de substances dégradables par voie enzymatique, comme la créatinine, dans des fluides biologiques, dans lequel les enzymes sont immobilisées sur une électrode de travail, **caractérisé en ce que** l'on dépose sur l'électrode de travail une enzyme avec une ou plusieurs substances tensio-actives en solution aqueuse et on la laisse sécher et que l'on immobilise chimiquement dessus au moins une deuxième enzyme dans une étape suivante.

**2.** Procédé selon la revendication 1, **caractérisé en ce que** l'on utilise comme substances tensio-actives des polyalcools et/ou des détergents, de préférence des agents tensio-actifs non ioniques.

**3.** Procédé selon la revendication 1 ou 2, **caractérisé en ce que** l'on immobilise la au moins deuxième enzyme par réticulation, liaison covalente ou inclusion dans une matrice.

**4.** Procédé selon la revendication 3, **caractérisé en ce que** l'on immobilise la au moins deuxième enzyme au moyen de glutardialdéhyde.

**5.** Procédé selon l'une des revendications 1 à 4, **caractérisé en ce que** l'on dépose une membrane de couverture après l'immobilisation.

**6.** Biocapteur avec électrode de travail, électrode de référence et contre-électrode, préparé au moyen du procédé selon l'une des revendications 1 à 5, **caractérisé en ce que** l'électrode de référence est une électrode Ag/AgCl et la contre-électrode, une électrode de carbone et que l'électrode de travail est constituée de carbone, de métal, d'oxydes métalliques ou d'un mélange de carbone et de métal ou d'oxydes métalliques, les électrodes étant déposées sur un substrat non conducteur.

**7.** Biocapteur selon la revendication 6 pour la détection de la créatinine, **caractérisé en ce que** de la sarcosinoxydase est adsorbée sur l'électrode de travail et que de la créatininase et de la créatinase sont immobilisées dessus.

**8.** Biocapteur selon la revendication 7, **caractérisé en ce qu'**il est construit à partir de deux systèmes à trois électrodes, lequel premier système d'électrodes avec les enzymes créatininase, créatinase et sarcosinoxydase sert à la détermination de la somme de créatinine et de créatine, et lequel deuxième système d'électrodes avec les enzymes créatinase et sarcosinoxydase sert à la détection de créatine, les deux résultats étant soustraits pour la détection de créatinine.

**9.** Biocapteur selon la revendication 8 qui comprend un autre système d'électrodes qui sert à l'élimination d'interférences électrochimiques.